# EUROPÄISCHE PATENTSCHRIFT

(11) **EP 3 226 953 B1**
(45) Veröffentlichungstag und Bekanntmachung des Hinweises auf die Patenterteilung: **26.03.2025**
(21) Anmeldenummer: 15823743.8
(22) Anmeldetag: 04.12.2015
(51) Int. Cl.: A61M 25/00, A61M 25/10, A61F 2/00

(54) **BLASENKATHETER FÜR DIE MINIMALE INVASIVE ABLEITUNG VON URIN**
BLADDER CATHETER FOR THE MINIMALLY INVASIVE DISCHARGE OF URINE
CATHÉTER URINAIRE POUR L'ÉVACUATION D'URINE LA MOINS INVASIVE POSSIBLE

(30) Priorität: 04.12.2014 DE 102014017873
(43) Veröffentlichungstag der Anmeldung: 11.10.2017
(73) Patentinhaber: Advanced Medical Balloons GmbH, 68753 Waghäusel (DE)
(72) Erfinder: GÖBEL, Fred, 69259 Wilhelmsfeld (DE)
(74) Vertreter: Küchler, Stefan
(86) Internationale Anmeldenummer: PCT/IB2015/002282
(87) Internationale Veröffentlichungsnummer: WO 2016/087926

(56) Entgegenhaltungen:
- WO-A1-2011/139498
- WO-A2-2007/005734
- DE-A1- 102011 110 778
- DE-A1- 3 742 710
- US-A- 5 306 226
- US-A- 5 441 485

## Beschreibung

Die Erfindung richtet sich auf eine Vorrichtung in Form eines Tubus oder Katheters zum Drainieren oder Verschließen eines natürlichen oder künstlichen Blasenausgangs, umfassend einen von extrakorporal bis in die Blase hinein applizierbaren Schaftkörper sowie einen jenen manschettenförmig umgebenden Ballon zur vesikalen Verankerung des Schaftkörpers in der Blase.

Blasenverweilkatheter zur kontinuierlichen, geschlossenen Ableitung von Urin aus der Blase eines Patienten sind in verschiedenster Ausführungsform Teil des Versorgungsstandards.

Blasenverweilkatheter werden in der Regel durch trans-urethrale Katheterisierung in die Harnblase eingebracht. Im Gegensatz zur Katheterisierung der Blase mit Einmalkathetern, bei der intermittierend ein urinableitender Katheterschaft durch die Harnröhre in das Blasenlumen vorgeschoben wird, sind Katheter zur Dauerkatheterisierung an ihrem distalen, im Inneren der Blase zu liegen kommenden Ende, typischerweise mit einem ballonartigen Ankerelement (Retentionsballon) ausgestattet.

Bei der Mehrzahl der Blasenverweilkatheter bestehen diese Retentionsballons aus elastisch dehnbarem Schlauchmaterial, welches eng angeschmiegt und ohne jede Präformation auf dem Schaftkörper des Katheters aufsitzt. Durch Befüllung mit einem in der Regel flüssigen Medium dehnt sich aus dem zylindrischen Schlauchstück, bei entsprechend Kraftaufwand, ein sich in der Regel sphärisch entwickelnder Ballonkörper aus. Nachteilig bei derartigen, unter hohem Fülldruck aufgedehnten Ankerballonen ist vor allem die sich einstellende harte Konsistenz des Ballons. Bei längerer Liegedauer sind im Auflagebereich des Ballons, im Bereich des sogenannten Blasen-Trigonums, nicht selten strukturelle Schädigungen der Blasenschleimhaut zu beobachten, die zum Teil in dauerhafte Ulzerationen übergehen und bei bakterieller Besiedlung zur Quelle chronischer Entzündung des Harntraktes werden können.

Die vorliegende Erfindung geht dabei aus von einer Katheterbauweise, die bereits in Teilen in der WO2008/038172 beschrieben ist.

Der vorgestellte "Indwelling urinary catheter with enlarged sealing surface" beschreibt einen bereits bei der Herstellung auf sein Arbeitsmaß vorgeformten Katheterballon, welcher dem Schaft des Katheters, in der Blase platziert, in nur inkomplett befülltem, schlaffem Zustand aufsitzt. Bedingt durch die spezifische, geringe Volumendehnbarkeit der Ballonhülle, kann sich der Ballon bei axial von außen auf den Ankerballon wirkender Zugkraft nicht in einem Umfang verformen, der sein Herausgleiten bzw. Schlüpfen durch die Harnröhre ermöglicht. Stattdessen geht der den Katheter im Trigonum der Blase ankernde Ballon bei Zugwirkung von außen in seine durch die Herstellung vorgegebene Form über. Er deformiert sich nicht, und entwickelt im Inneren einen Druck, der in etwa der jeweils auf ihn wirkenden Zugkraft entspricht. Bei nachlassender Zugwirkung geht der Druck im Balloninneren auf den in der Blase herrschenden physiologischen Druck zurück. Das vorgeschlagene Design ermöglicht so eine nahezu druckneutrale Wirkung des Ballons auf das ihm anliegende Gewebe. Läsionen, wie sie durch konventionelle, prall gefüllte Ballonkörper verursacht werden, können bei der beschrieben Bauweise weitgehend ausgeschlossen werden.

Ein Problem, welches mit der Anlage von Blasenverweilkathetern assoziiert ist, sind aufsteigende Infektionen bzw. Besiedlungen des Blasenbinnenraumes durch Keime, welche die Blase über die Harnröhre des Patienten, quasi der unphysiologischen Oberfläche des Katheterschaftes folgend, nach einigen Tagen Katheterliegezeit erreichen.

Zwar ermöglicht die in der WO2008/038172 beschriebene druckneutrale Dichtung des Blasenausgangsbereichs einen verbesserten Schutz vor bakteriell besiedelten Läsionen bzw. Ulzerationen der Blasenwandung, das Problem der über die Harnröhre zur Harnblase aufsteigenden Infektionen bei dauerkatheterisierten Patienten wird jedoch nicht zufriedenstellend gelöst.

Die US 5,441,485 beschreibt ebenfalls eine Kathetervorrichtung gemäß Oberbegriff des Anspruchs 1.

Es ist demgemäß das Ziel der vorliegenden Erfindung, Blasenverweilkatheter für die dauerhafte Platzierung in der Harnblase dahingehend zu verbessern, dass das Besiedlungsrisiko der Blase mit Keimen insgesamt reduziert werden kann.

Hierzu sollen zum Einen die in der Blase vom ankernden Ballon auf die Blasenwandung übertragenen Kräfte minimiert bzw. die Kontaktfläche des ankernden Ballons zur Blasenwandung derart gestaltet werden, dass durch punktuelle Kraftwirkung verursachte Läsionen, wie sie bei herkömmlichen Kathetern typisch sind, weitestgehend vermieden werden können.

Ferner soll eine deutlich verbesserte Dichtung des Spaltraumes zwischen dem urinableitenden Schaft des Katheters und der Schleimhaut der Harnröhre erreicht werden.

Die vorgestellten Katheter-Vorrichtungen zur kombinierten Dichtung des Blasen-Trigonums und der Harnröhre werden bevorzugt anstatt mit flüssigen Medien, vorzugsweise mit Luft befüllt. Die Fülldrucke liegen dabei im niedrigen Millibar-Bereich und überschreiten den in der Blase des Patienten herrschenden physiologischen Druck nicht, sind also in ihrer Wirkung konzeptionell druckneutral.

Zur Vermeidung aufsteigender Infekte wird bei der vorliegenden Erfindung das proximale Ende des den Katheter in der Blase ankernden Retentionsballons in die Harnröhre hinein oder durch sie hindurch verlängert.

Der bei konventioneller Katheterisierung zwischen Katheterschaft und Harnröhrenwandung entstehende Spalt füllt sich in der Regel mit stark keimhaltigen Sekreten. Bei der erfindungsgemäß vorgeschlagenen Tamponade des Harnröhrenlumens durch einen vorzugsweise mikrodünnwandigen urethralen Ballonfortsatz, wird der residuale Spaltraum zwischen Katheterschaft und Harnröhre weitestgehend verschlossen. Die dichtende "Tamponade" des Harnröhrenlumens erfolgt hierbei durch weitgehend neutral wirkende Kräfte. Der in der Blase herrschende intra-abdominelle Druck wird vom vesikalen Anteil des Ballonkörpers aufgenommen und für den raumfüllenden Verschluss des Harnröhrenlumens verwendet. Im Rahmen der bevorzugten Anwendung der Erfindung sind also lediglich im Körper herrschende, physiologische Kräfte wirksam.

Der vesikale Anteil des erfindungsgemäßen Ballonköpers wird bevorzugt zwiebelförmig oder konisch ausgeführt, wobei die Spitze der Zwiebel bzw. des Konus zum Trigonum der Blase gerichtet ist, um sich so, im partiell befüllten Zustand, bestmöglich dem jeweiligen Situs des Blasenausgangs anzuschmiegen. Der urethrale Anteil des Ballonkörpers wird erfindungsgemäß mit einem "residualen" Durchmesser präformiert, der den Durchmesser des Harnröhrenlumens um das ca. 0,5 bis 1,5 fache, bevorzugt um das 0,5 bis 1,0 fache überschreitet. Der so im Durchmesser überdimensionierte urethrale Ballonanteil legt sich damit der Harnröhrenwandung unter Ausbildung einer radial orientierten Faltung der residualen Hülle an. Kaliberschwankungen des Harnröhrenlumens können durch den residualen Durchmesser über den Längsverlauf der Harnröhre hinweg ausgeglichen werden. Durch das Übermaß wird sichergestellt, dass alle Harnröhrenanteile tamponiert werden können, ohne dass hierzu eine kraftintensive Aufdehnung der Ballonhülle erforderlich ist. Der erfindungsgemäße, ankernde und dichtende Ballon wird bevorzugt durch ein inkomplettes bzw. partielles Füllvolumen befüllt, welches derart bemessen ist, dass der Ballonkörper als Ganzes in einem schlaffen Dehnungszustand verbleibt, die Ballonwandung also keiner permanent wirkenden, dehnenden Kraft ausgesetzt ist.

Als alternative Ausführung der vorliegenden Erfindung welche jedoch nicht Gegenstand der Patentansprüchen ist, kann der urethrale Anteil des Ballonkörpers derart bemessen werden, dass er den Durchmessermaßen der jeweiligen Harnröhre entspricht oder diese leicht unterschreitet. Die urethrale Ballonhülle geht bei Befüllung des Ballons, im Gegensatz zur residual bemessenen Ausführung, in eine vollständig Aufrichtung ohne Ausbildung radialer Einstülpungen über.

Der Ballonkörper kann alternativ zur partiellen Befüllung auch mit einem Volumen befüllt werden, dass dem Volumen des frei ausgeformten Ballons entspricht oder dieses leicht überschreitet.

Die Befüllung der erfindungsgemäßen Katheter erfolgt vorzugsweise mit einem gasförmigen Medium bzw. mit Luft.

Bevorzugt ist dabei der ankernde und dichtende Ballon mit dem vorzugsweise gasförmigen Füllmedium in einer derartigen Menge befüllt, dass es das Volumen des frei ausgeformten Ballons mit einem höheren Druck ausfüllt als dem atmosphärischen Druck.

Die Zuleitung des Füllmediums erfolgt bevorzugt über eine in die Wandung des ballontragenden Schaftes integrierte Zuleitung. Alternativ kann die Befüllung auch über eine Zuleitung erfolgen, die direkt in das proximale Ende des Ballonkörpers mündet, also nicht in den Katheterschaft intergiert ist. Die Zuleitung kann ebenso durch den Spalt zwischen dem proximalen Schaftende des Ballons und der äußeren Fläche des Schaftes hindurchgeführt werden. Die Zuleitung verfügt vorzugsweise über ein endständiges Einwegeventil, das sich beim Aufsetzen einer Füllspritze öffnet und beim Entfernen des Spritzenkonus selbständig verschließt. Die volumendefinierte Befüllung mit einer Füllspritze sichert die bevorzugte Füllung des Katheterballons in einen inkomplett befüllten, spannungslosen Zustand. Bei einer kompletten Befüllung oder einer Befüllung über das frei entfaltete Ballonvolumen hinaus, kann sich der in situ einstellende Fülldruck vom Anwender bei Bedarf leicht manometrisch kontrolliert werden.

Die Zuleitung kann ferner eine fix integrierte, manometrisch wirkende Druckanzeige aufweisen, die das fortlaufende Monitoring des herrschenden Fülldrucks ermöglicht. Eine derartige Anzeige ermöglicht beispielsweise die Feststellung relativer Veränderungen des im Blasenbinnenraum herrschenden intra-abdominellen Drucks.

Bei Verwendung angemessen dünnwandiger Ballonmaterialien unterbindet die sich im urethralen Anteil des proximal verlängerten Ballons einstellende Faltung, einen freien Abfluss von Urin, selbst bei niedrigsten beaufschlagten Fülldrucken, wirksam aus. Die Dichtung ist insbesondere bereits bei Normalwerten des intra-abdominellen Druckes gegeben, also in der Größenordnung von rund 10 mbar bzw. 5 bis 7 mmHg. Wie zuvor ausgeführt, kann der erfindungsgemäße Katheter, bei entsprechender inkompletter Befüllung, den diagnostisch und therapeutisch wichtigen intra-abdominellen Druck mit guter absoluter Näherung im Blaseninneren aufnehmen.

Die weitgehende Elimination sekret-akkumulierender Räume in der erfindungsgemäß tamponierten Harnröhre wirkt sich zudem vorteilhaft auf die Effizienz optionaler antibakterieller Beschichtungen auf der Ballonoberfläche aus. Zum einen wird die Menge des keimhaltigen Sekretes reduziert, zum anderen wird der verbleibende Sekretfilm einer residual vergrößerten, antibakteriell wirksamen Oberfläche ausgesetzt.

Die Verwendung maximal dünnwandiger, bevorzugt durch Blasformung aus vorextrudiertem Schlauchmaterial hergestellten Ballonelementen, ermöglicht hervorragend ebene Oberflächen, die die kolonisationsrelevante Gesamtoberfläche des Ballonkörpers optimal reduzieren. Möglich wird dies durch die bei der Formung auf den Rohschlauch wirkende hohe radiale und axiale Streckung, die Unebenheiten der Wandung optimal nivelliert und bei der Ausdünnung in den mikrodünnen Bereich zu nahezu perfekt ebenen Oberflächen führt. Für die Erfindung werden daher extrem dünnwandige PUR Ballons favorisiert.

Neben der größtmöglichen Reduktion keimhaltiger Reservoirs zwischen Harnröhre und Katheterschaft, gestattet die erfindungsgemäße Fortsetzung des proximalen Ballonendes in die Harnröhre hinein oder durch die Harnröhre hindurch einen verbesserten Schutz vor direkter mechanischer Irritation bzw. Läsion des Epithels durch den Katheterschaft, wie diese bei der herkömmlichen Dauerkatheterisierung nicht ungewöhnlich sind.

Die erfindungsgemäße Dichtung des Harnröhrenlumens durch einen flächig zum Harnröhrenepithel hin dichtenden, dünnwandigen Ballon erlaubt ferner besonders kleinlumige, atraumatische Ausführungen des drainierenden Schaftanteils der Kathetervorrichtung. Der Schaft kann beispielsweise aus Polyurethan (PUR) gefertigt sein, und wird in diesem Fall bevorzugt aus Materialien der Shore Härten 60A bis 90A hergestellt, besonders bevorzugt aus Materialien des Härtebereiches 70 bis 85A. Der Außendurchmesser des drainierenden Schaftes beträgt bevorzugt 2 bis 6 mm, besonders bevorzugt 3 bis 5 mm. Die Wandungsstärke beträgt bei Verwendung der genannten PUR-Härten bevorzugt 0.1 bis 0.5 mm, besonders bevorzugt 0.15 bis 0.3 mm.

Zur weiteren Reduktion des Schaftaußendurchmessers des urinableitenden Schaftelementes, kann die Befüllung des Ballonkörpers durch einen am proximalen Ende des urethralen Ballonfortsatzes hergestellten direkten Zugang zum Ballon erfolgen. Auf eine schaft-integrierte Zuleitung zur Befüllung des Ballons kann somit im Sinne eines möglichst großen Drainagelumens bei möglichst kleinem Schaftaußendurchmesser verzichtet werden.

Neben Ballonkörpern, die aus einer einzigen durchgängigen Ballonhülle bestehen, können Ballonkompartimente, die sich nach proximal an einen vesikalen Ballon anschließen auch aus separat ausgeformten Ballonelementen aufgebaut sein, die sich unmittelbar sequentiell aneinanderreihen. Diese sind bevorzugt miteinander verbunden und erlauben eine kommunizierende Befüllung der jeweiligen Kompartimente.

Von besonderer Bedeutung bei der Vermeidung bakterieller Infekte in der Blase eines dauerkatheterisierten Patienten sind ferner Ansammlungen von Urin, die sich um einen, in den Blasenbinnenraum hineinreichenden vesikalen Ballonanteil ansammeln bzw. sich unterhalb der distalen Abflussöffnung des Katheterschaftes "poolen". Die Erfindung schlägt zur Vermeidung solcher "Sumpf-Bildungen" bzw. zur Vermeidung von Restharn einen möglichst flach diskoiden Auftrag des vesikalen Ballonanteils auf dem Blasenboden vor, der zudem möglichst mit einer trichterartigen, in den distalen Ballonradius invertierten Mündung versehen ist, die bevorzugt 3 bis 8 mm unter den distalen Ballonradius reicht, bzw. dort in den urinableitenden Katheterschaft übergeht. Der Übergang in den ableitenden Schaft soll konzeptionell bevorzugt auf der Höhe des Übergangs des Blasenbodens in die Harnröhre liegen. Die trichterartige Inversion soll derart ausgeformt sein, dass sie auch bei axialem Zug am Katheterschaft offen bleibt und sich die distalen Ballonportionen nicht über dem Trichterboden schließen. Die Trichtermündung kann optional durch ein die Trichterform stabilisierendes, entsprechend ausgeformtes Folienelement verstärkt sein.

Ferner liegt es im Rahm,en der Erfindung, dass die distale Mündung ^{™} des Schaftkörpers proximal des distalen Endes des Ballons liegt.

Zur Optimierung des Herstellaufwandes eines erfindungsgemäßen Katheters sind ferner Ausführungsformen möglich, bei denen sowohl der Schaft als auch der Ballon aus einem einzigen Materialrohling ausgeformt werden. Hierbei wird konzeptionell ein Ballonende durch das andere gestülpt. Das im Rahmen der Formung entstehende ableitende Schaftelement kann durch eine wellige Korrugation hinsichtlich seiner radialen Stabilität und knickfreien Verbiegbarkeit vorteilhaft optimiert werden.

Erfindungsgemäß kann weiterhin vorgesehen sein, dass der distale Anteil des Ballons derart ausgeformt ist, dass er den Maßen einer Prostata-Resektionshöhle (PH) entspricht und vorzugsweise mit einem diesbezüglich residual dimensionierten Durchmesser versehen ist.

Für diesen Anwendungsfall sieht die Erfindung optional weiter vor, dass das distale Ende des in der Prostata-Resektionshöhle (PH) platzierten Ballons nicht in die Blase hineinragt, sondern etwa bündig zu dem Blasenboden verläuft.

Schließlich entspricht es der Lehre der Erfindung, dass die Länge des urethralen Ballonsegments zwischen 3 und 10 cm liegt, vorzugsweise zwischen 5 und 8 cm.

Weitere Merkmale, Eigenschaften, Vorteile und Wirkungen auf der Basis der Erfindung ergeben sich aus der folgenden Beschreibung einiger Ausführungsbeispiele der Erfindung sowie anhand der Zeichnung. Hierbei zeigt:
- Fig. 1: eine Ausführungvariante der Vorrichtung bei trans-urethraler Anlage in der männlichen Harnröhre;
- Fig. 2a: eine bevorzugte Ausführungsform in freier Ballonentfaltung, wobei der Ballonkörper im Übergang vom intra-vesikalen in das trans-urethrale Segment eine konische bzw. kegelartige Form aufweist und der urethrale Anteil bis in den oberen Harnröhrenanteil reicht;
- Fig. 2b: eine besondere Ausführungsform des Ballonkörpers bei freier Ballonentfaltung, wobei der Ballonkörper keine Auflagefläche im Bereich des Blasenbodens bzw. -trigonums formiert, und sich stattdessen innerhalb einer Prostata-Resektionshöhle positioniert, und von dort in einen urethral oder trans-urethral positionierten Ballonanteil übergeht;
- Fig. 2c: einen horizontalen Schnitt eines in Fig. 2b beschriebenen Ballonsegmentes zur Tamponade einer chirurgisch erzeugten Prostataloge;
- Fig. 3a: eine ein-lumige Schaftvariante, wobei das trans-urethrale Segment des Ballonkörpers über die äußere Harnröhrenöffnung hinaus reicht und vom proximalen Ende des Ballonkörpers her direkt befüllt wird;
- Fig. 3b: eine abgewandelte Ausführungsform der in Fig. 3a beschriebenen Vorrichtung, wobei das proximale, über das äußere Orificium hinausreichende Ballonende durch ein hülsen- oder scheibenartiges Element fixiert oder gefasst werden kann;
- Fig. 3c: eine weitere Ausführungsform der in Fig. 3a beschriebenen Vorrichtung;
- Fig. 4a: eine Ausführungsvariante des Katheters für die trans-urethrale Dichtung der weiblichen Harnröhre;
- Fig. 4b: eine Abwandlung der in Fig. 4a beschriebenen Vorrichtung, mit konischer Ausführung des urethral tamponierenden trans-urethralen Ballonsegmentes;
- Fig. 5: eine besondere Ausführung eines Katheterschaftes, wobei durch faltenbalgartige Korrugation des Schaftes kristalline Auflagerungen auf den inneren und äußeren Schaftoberflächen vermieden werden können;
- Fig. 6: eine besonders kostengünstige Ausführung eines Katheters, der aus einem einzigen, durchgängig ausgeformten Element aufgebaut ist.

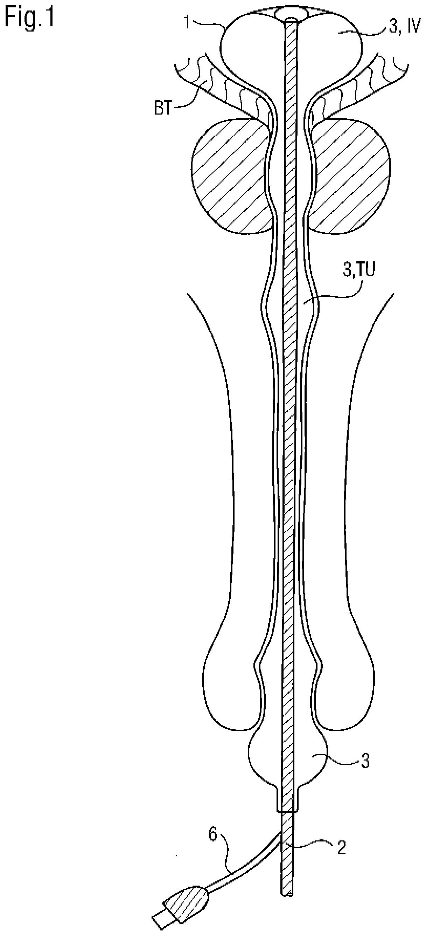

Fig. 1 zeigt eine Schnittbild durch ein einen männlichen Urethraltrakt, mit einer erfindungsgemäßen, trans-urethral platzierten Vorrichtung 1 in Form eines Blasenverweilkatheters mit einem Katheterschaft. Dessen Schaftkörper 2 trägt bei dieser Ausführungsform über seine gesamte vesikale und urethrale Ausdehnung einen Ballon 3, welcher den Schaftkörper 2 manschettenförmig umgibt. Vorzugsweise ist der Ballon 3 in allen seinen Abschnitten - sowohl in seinem vesikalen Bereich als auch in seinem urethralen Bereich - vollständig auf das zur spannungslosen Tamponade des betreffenden Hohlorgans erforderliche Arbeitsmaß ausgeformt. Erfindungsgemäß überschreitet der Durchmesser in seinem urethralen Bereich den Durchmesser der Urethra residual. Der ankernde und dichtende Ballon 3 weist in seinem intra-vesikalen Anteil IV eine zwiebelförmige oder konisch-kegelartige Erweiterung auf. Erfindungsgemäß bevorzugt werden Formgebungen des Ballons, die es gestatten, dass der Ballon sich möglichst großflächig dichtend in die Auslassportion der Blase fügt, und dort so, neben einer möglichst gleichförmigen und möglichst atraumatisch wirkenden Kraftexposition auf das Blasentrigonum BT, eine möglichst effiziente Dichtung bei möglichst geringem wirkendem Fülldruck ermöglicht.

Das urethral-seitige Ende des Ballonelementes 3 geht in einen verschlankten, urethralen Fortsatz TU über. Wie hier dargestellt, kann sich der urethrale Fortsatz über die gesamte Länge der Harnröhre, bis über das äußere Ostium hinaus erstrecken.

Das Durchmesserverhältnis der vesikalen Erweiterung IV zum urethralen Fortsatz TU liegt bevorzugt bei 2 : 1 bis 6 : 1, besonders bevorzugt bei 3 : 1 bis 4 : 1. Die Wandungsstärke beträgt im Bereich der intra-vesikulären Erweiterung IV bevorzugt 5 bis 30 Mikrometer, besonders bevorzugt 10 bis 15 Mikrometer. Im trans-urethralen Segment TU weist der Ballon bevorzugt 10 bis 30 Mikrometer, und besonders bevorzugt 12 bis 20 Mikrometer auf.

Die Befüllung des Ballonelementes 3 erfolgt bei der dargestellten Ausführung durch einen Befüll-Kanal, der in den Katheterschaft integriert ist und am proximalen Schaftende in eine schlauchartige Befüll-Zuleitung 6 übergeht. Zur weitest möglichen Reduktion der Fülldrucke bzw. der durch die Vorrichtung auf die anliegenden Gewebe ausgeübten Kräfte, wird der Ballonkörper nur inkomplett, mit beispielsweise 60 bis 80% seines frei ausgeformten, nicht mit Druck beaufschlagten Volumens befüllt. Die konzeptionell bevorzugte, schlaffe bzw. spannungslose Beschaffenheit des so befüllten Ballons 3 erlaubt intra-vesikulär ein optimiert dichtendes Anschmiegen der schlaffen Ballonhülle an die individuell ausgeprägte Wandung der Ausgangsportion der Blase. Der intra-vesikulär vom schlaff befüllten Ballon 3 aufgenommene Druck wirkt im urethralen Segment TU des Ballons 3 flächig dichtend. So kann im idealen Fall ein dichtender Verschluss der unteren Harnwege bei insgesamt nahezu druckneutraler Platzierung des Katheters ermöglicht werden.

Die Befüllung des Ballons 3 kann sowohl durch gasförmige als auch durch flüssige Medien erfolgen.

Gasförmige Medien, wie Raumluft, bieten bei der beschriebenen bevorzugten Ausführung des Ballonelementes 3 in Polyurethan den weiteren Vorzug der gewebeschonenden Kompressibilität des Mediums. Sie können zudem, im Gegensatz zu Flüssigkeiten, einfach appliziert und bei Bedarf auch mit Hilfe eines Druckreglers überwacht und korrigiert werden.

Der urethrale Fortsatz TU kann direkt aus dem vesikalen Segment 4 des Ballons 3 hervorgehen, kann aber auch als strukturell eigenständiges Kompartiment in proximaler Verlängerung an den vesikalen Ballon angefügt werden. Das vesikale Ballonsegment 4 und das sich urethral anschließende Ballonsegment 5 sind dann miteinander kommunizierend verbunden.

Bei einer derartigen sequentiellen Anordnung können optional auch verschiedene Materialien kombiniert werden. So lassen sich bei Bedarf im urethralen Ballonanteil 5 auch extrem dünnwandige Wandstärken bis in den Bereich von 5 bis 10 Mikrometer herstellen, welche die urethrale Dichtungsleistung über die bei Formung aus einem einzigen Rohling hinaus erreichbare wandstärkenkorrelierte Dichtung entsprechend verbessern.

Neben Polyurethan kann das Ballonelement 3 aus vergleichbar dünnwandigen Weichfolien alternativer Materialien, wie beispielsweise Polyethylen, Polyvinylchlorid oder auf TPE basierten Grundstoffen gefertigt werden. Bevorzugt werden jedoch die spezifischen Eigenschaften von Polyurethanen der Härten Shore 70A bis 95A, besonders bevorzugt Shore 85A bis 90A. Die Herstellung erfolgt bevorzugt durch Blasformung aus vorextrudiertem Schlauchmaterial. Für die Blasformung sind ferner koextrudierte Ausgangsmaterialien denkbar, die beispielsweise PUR und PVC in koaxial extrudierter Weise kombinieren.

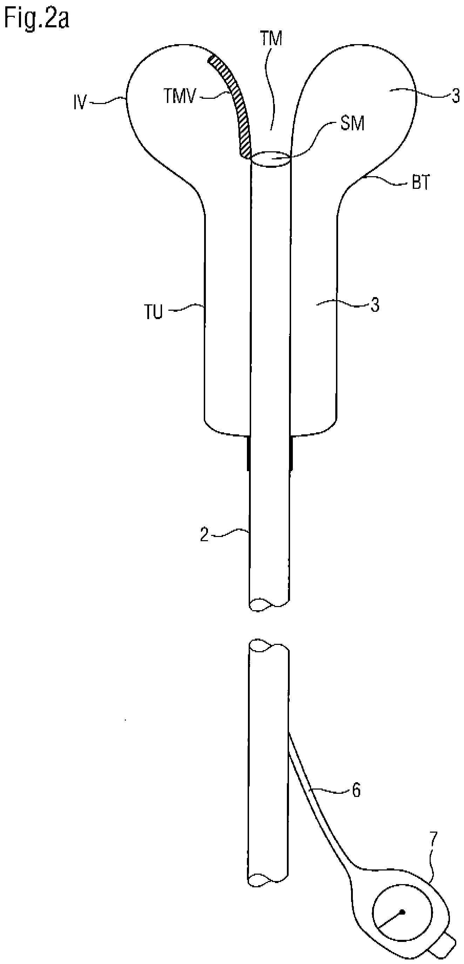

Fig. 2a stellt einen bevorzugt geformten Ballonkörper 3 in freier Entfaltung dar. Der intra-vesikale Anteil IV des Ballons 3 ist hier im Übergangsbereich zum trans-urethralen Fortsatz TU des Ballons 3 hin konisch verjüngt. Der trans-urethrale Fortsatz TU erstreckt sich bei dieser besonderen Ausführung für die männliche Harnröhre nur bis zum Übergang des oberen Drittels zum mittleren Drittel der Harnröhre.

Das distale Ballonende ist bevorzugt mit einer trichterartigen Einziehung bzw. Inversion TM versehen, deren Mündung SM sich um befüllten und vesikal platzierten Zustand etwa auf Höhe des Übergangs bzw. Blasentrigoniums BT einstellt. Die Stabilität der Trichterformation kann durch ein separates, trichterartig ausgeformtes Element TMV aus beispielsweise versteifender Folie stabilisiert werden.

Die Befüllzuleitung 6 zum Katheterballon ist an ihrem freien Ende hier mit einem, den Fülldruck anzeigenden Anzeigemechanismus 7 versehen, der es erlaubt auf einfache Weise Veränderungen im vesikulären, und damit weitgehend analog, im intra-abdominellen Druck festzustellen. Hierzu wird der Ballon bevorzugt mit einem inkompletten Füllvolumen befüllt, welches den Ballon in einen schlaffen, nicht gedehnten Zustand bringt, der eine präzise Aufnahme der jeweils vesikal bzw. intraabdominell herrschenden Drucke erlaubt. Die Vorrichtung kann so als apparativ einfache Alternative zu komplex aufgebauten Systemen zur Messung des absoluten intra-abdominellen Drucks verwendet werden bzw. relative Veränderungen des Drucks im Abdomen anzeigen.

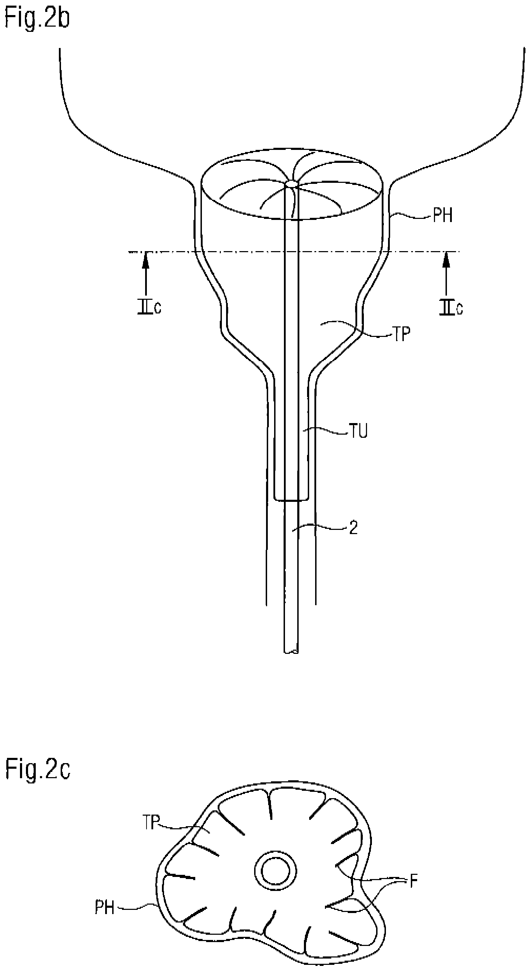

Fig. 2b zeigt eine Ausführungsform des Ballonkörpers 3, bei der der Ballonkörper keine besondere Auflagefläche im Bereich des Blasenbodens bzw- Trigonums formiert, sich stattdessen innerhalb einer Prostata-Resektionshöhle PH positioniert, und von dort in ein urethral positioniertes Ballonsegment TU übergeht. Der für die Platzierung in der sich nach Resektion der Prostata einstellenden Kavität ausgeformte Ballonanteil TP schmiegt sich raumfüllend an den Situs an. Der Ballonanteil TP ist dabei in seinen Formungsmaßen bevorzugt derart dimensioniert, dass er die Maße der jeweiligen Prostata-Resektionshöhle überschreitet und der Wandung der jeweiligen Kavität optimal spannungslos und gleichzeitig optimal dichtend in Faltung anliegt.

Diese Ausführungsform ist vor allem für Patienten vorteilhaft, die resektionsbedingt eine Verschlussinsuffizienz der Blase aufweisen bzw. bei denen permanent Urin in die Resektionshöhle vordringt und von dort in die Harnröhre abläuft. Um die spannungslose Schmiegung der Ballonhülle in der Prostatahöhle zu optimieren, kann der Ballonanteil TP mit einem axial angelegten, faltenbalgartigen Profil TPF versehen werden. Im Durchmesser irregulär geformte Resektionshöhlen können so besonders vorteilhaft, bei möglichst homogener Kraftwirkung auf die dem Ballon anliegenden Strukturen, tamponiert werden. Sollte neben dem internen Sphinkter der Blase auch der externe Schließmuskel betroffen sein, kann die so bedingte Verschlussinsuffizienz durch den urethralen Fortsatz TU des Ballonkörpers, welcher über die Strukturen des Beckenbodens hinaus verlängert ist, effizient dichtend verschlossen werden.

Fig. 2c zeigt die Faltung F der residual dimensionierten Ballonhülle TP, die sich bei Positionierung in der Prostata-Höhle einstellt im transversalen Schnitt. Insbesondere bei in die Loge hineinragenden prominenten Strukturen bzw. Anteilen der Höhlenwandung kann durch die residuale Ballonbemaßung eine gleichförmig wirkende, effizient dichtende Tamponade der Resektionshöhle hergestellt werden.

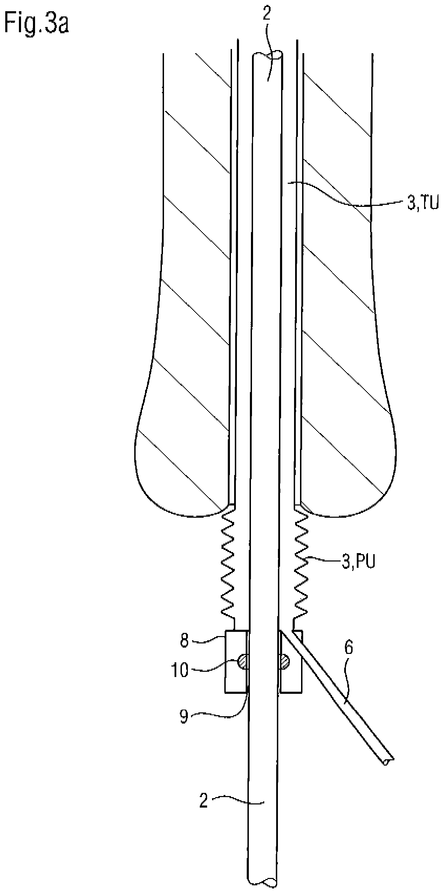

Fig. 3a beschreibt eine Ausführungsform der Erfindung, bei der das proximale Ende des urethralen Ballonsegmentes PU über das äußere Ende der Harnröhre hinausragt. Das Ballonelement 3 wird hier durch eine Zuleitung befüllt, die direkt mit dem extrakorporalen, proximalen Ende des urethralen Ballonsegmentes PU verbunden ist. Die Ballonhülle schließt hier nach proximal durch ein separates, den Ballon 3 nach proximal abschließendes Element 8 ab, welches in das proximale Hüllenende PU eingeklebt wird. Das Abschlusselement 8 weist eine Öffnung 9 auf, die den urinableitenden Katheterschaft 2 dicht schließend aufnimmt. Die Öffnung 9 kann wiederum mit einem lippenartig oder ringartig dichtenden Element 10 versehen sein, die das Abschlusselement samt daran befestigtem prä-urethralen Ballonende PU, relativ zum Schaft 2 verschiebbar macht. Das über die Harnröhrenöffnung hinausreichende Ballonende des urethral tamponierenden Ballonanteils kann so in faltenbalgartiger Weise zur Glans hin gestaucht bzw. in seiner Länge eingestellt werden.

Alternativ kann im Bereich der proximalen Ballonhülle auch ein für den Anschluss einer Schlauchzuleitung geeigneter Fortsatz der Ballonhülle selbst ausgeformt werden.

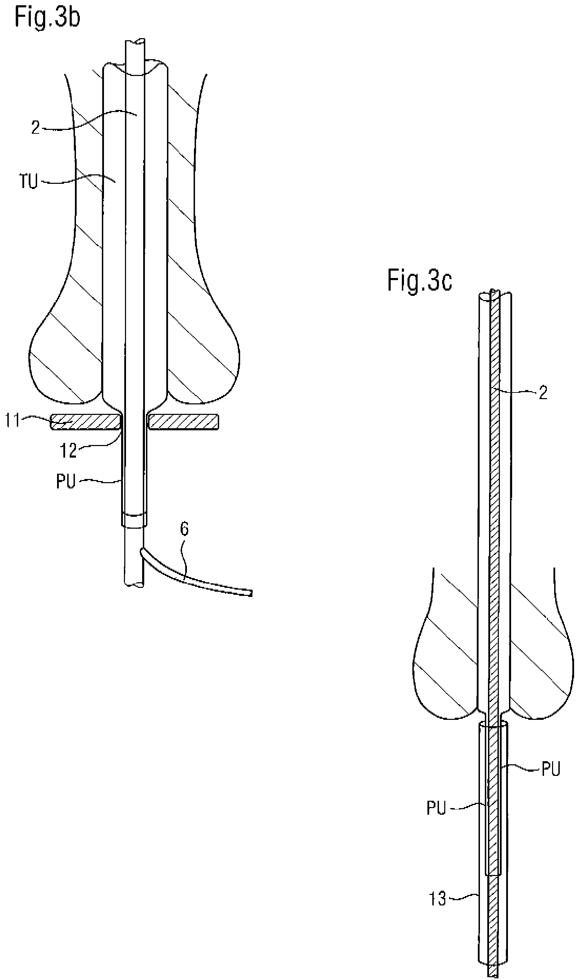

Fig. 3b und 33c zeigen zwei alternative Ausführungsformen hinsichtlich der Fixierung bzw. Ablängung des prä-urethralen Anteils des tamponierenden Ballons 3. Zum einen kann gemäß Fig. 3b das proximale Ballonende durch eine aus elastischem Material bestehende Scheibe 11 mit einer zentralen Öffnung 12 versehen sein, welche mit dicht schließender Spannung auf dem Katheterschaft 2 aufsitzt. Die Scheibe 11 begrenzt durch ihre pressende Wirkung auf den Schaft die prä-urethrale Ausdehnung des tamponierenden Ballons bzw. verhindert eine Befüllung des Ballonanteils proximal der Scheibe. Gemäß Fig. 3c kann ein hülsenartig verlängertes Element 13 in die Vorrichtung integriert sein, welches vom proximalen Schaftende her über das prä-urethrale Ballonende PU geschoben wird und so die Entfaltung der Ballonhülle vor der Harnröhrenöffnung flächig begrenzt.

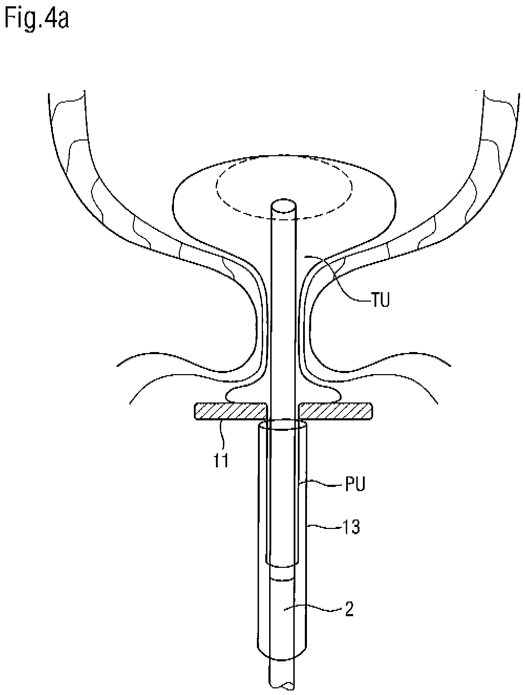

Fig. 4a zeigt eine speziell verkürzte Ausführung der urethralen Tamponade TU für die weibliche Harnröhre. Das urethrale Segment TU reicht hier bevorzugt durch die Harnröhre hindurch. Es weist eine Länge von 2 bis 6 cm, bevorzugt von 3 bis 4 cm auf. Das proximale Ende des trans-urethralen Segmentes TU geht bevorzugt in eine sphärische oder diskoide Erweiterung der Ballonhülle 14 über, die sich unmittelbar vor der äußeren Harnröhrenöffnung entfaltet.

Wegen der interindividuell variablen Länge der Harnröhre wird diese Ausführung bevorzugt mit einem erfindungsgemäßen Element 11 und/oder 13 ausgestattet, welche, wie in Fig. 3a gezeigt, eine prä-urethrale Ablängung und Fixierung der Ballonhülle erlaubt.

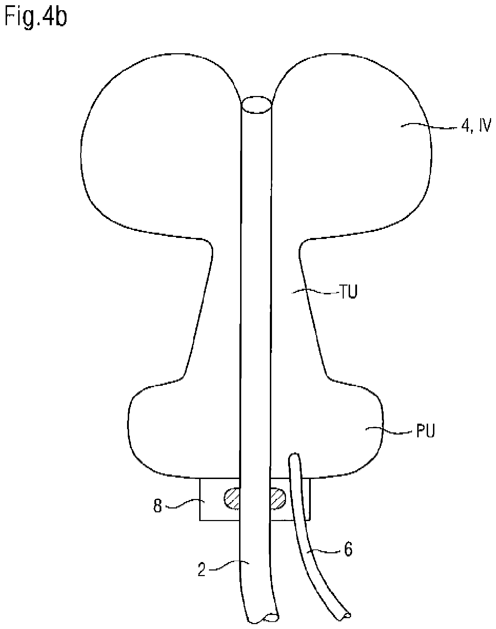

Da die weibliche Harnröhre in der Regel relativ ähnliche Längen, aber vom jeweiligen Sphinktertonus abhängige, variable Durchmesser aufweist, schlägt die Erfindung, wie in Fig. 4b gezeigt, eine vom inneren Harnröhreneingang zum äußeren Harnröhrenausgang hin reichende, konische Aufweitung des Durchmessers des trans-urethralen Segmentes TU vor.

Bei den Ausführungen nach Fig. 4a und 4b kann vorteilhaft das in Fig. 3a beschriebene, relativ zum Schaftkörper 2 verschiebbare Abschlusselement 8 zur Aufnahme des proximalen Endes des prä-urethralen Segmentes PU verwendet werden. Es erlaubt die längenanpassende Stauchung des extrakorporalen Endes zum Orificium hin, wobei das Element 8 vorteilhaft in das prä-urethrale, sphärisch oder diskoid erweiterte Segment eintaucht und so vor dem direktem Kontakt mit dem empfindlichen Orificium geschützt ist. Die Elemente 11 und/oder 13 zur Fixierung bzw. Ablängung des extra-korporalen Ballons können ebenfalls mit der Ausführung kombiniert werden.

Die in Fig. 4a und 4b beschriebenen Ausführungsformen können vorteilhaft auch für sog. supra-pubische Katheter zur Anwendung kommen, bei denen der trans-kutane Stichkanal in entsprechender Weise durch einen sich an den vesikalen Ballonanteil anschließenden Ballonfortsatz tamponiert werden kann. Ebenfalls können hier die erfindungsgemäß beschriebenen Fixier- und Ablängungstechniken des extrakorporalen Ballonsegmentes zur Anwendung kommen.

Ferner können die in Fig. 4a und 4b beschriebenen Ausführungsformen als Harnröhren-Verschlussvorrichtung bei funktionell insuffizienten Schließmuskeln bzw. Harninkontinenz zur Verwendung kommen. Das drainierende Schaftelement kann in diesem Falle durch ein blindes Röhrchen ersetzt werden, welches insbesondere derart knickstabil ausgeführt ist, dass es die Selbst-Katheterisierung durch den Patienten erlaubt. Bei entsprechend dünnwandiger Ausführung im niedrigen Mikrometerbereich der Ballonkörper, von beispielsweise 5 bis 15 µm, können minimal irritierende, gut trageverträgliche stöpselartige Verschluss-Produkte, insbesondere für stress-inkontinente Frauen hergestellt werden.

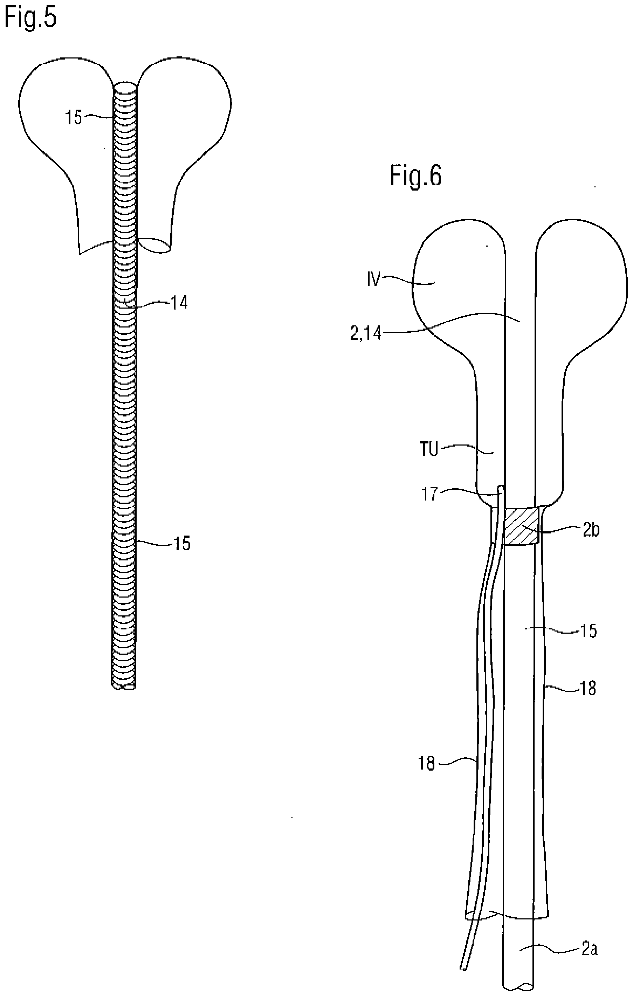

Fig. 5 zeigt eine besondere Ausführung eines aus elastischem Material gefertigten, urinableitenden Katheterschaftes 14, dessen Wandung mit einem wanddurchgängigen, wellartigen Profil 15 versehen ist, das dem Schaft eine faltenbalgartige Stauchungs- bzw. Streckungsmechanik verleiht. Durch intermittierende axiale Streckung des faltenbalgartigen Schaftkörpers 2 können kristalloide Urinablagerungen, die bei herkömmlichen urinableitenden Kathetern im Verlauf der Drainage zur progressiven Lumeneinengung führen, in lumeneröffnender Weise abgesprengt bzw. von der sowohl Innenwandung als auch Außenwandung des Katheters gelöst werden. Die besondere elastische Verformungseigenschaft des Schaftes führt bei nachlassender Zugwirkung zur prompten Rückstellung des Faltenbalgprofils. Die durch axiale Zugwirkung ausgelöste axiale Längenzunahme des Schaftes stellt sich vorzugsweise bereits bei geringen Relativbewegungen zwischen Patient und Katheter bzw. bei geringen Zugkräften ein.

Fig. 6 stellt eine Ausführung der urinableitenden Vorrichtung vor, deren Anteile nahezu vollständig aus einem einzigen Materialrohling, bevorzugt durch Blasformung, ausgeformt sind. Hierbei wird das Ende des ausgeformten Ballonkörpers 2a durch das andere Ende 2b des Ballons gestülpt. Das Ende 2b wird dann mit der Oberfläche des zum ableitenden Schlauch verlängerten Endes 2a dicht schließend verbunden. Hierdurch entsteht der erfindungsgemäße, ankernde und dichtende Ballonkörper mit vesikalem IV und trans-urethralem Anteil TU. Um den urinableitenden Schaftanteil 2 ausreichend knickstabil und biegbar zu gestalten, wird der Schaft, über seine ganze Länge oder auch nur in Anteilen, mit einer welligen Korrugation 15, in Art eines Wellschlauchs, versehen. So können beispielsweise für die Ballonausformung vorteilhafte Polyurethan-Typen höherer Durometer der Spanne Shore 90A bis 95A sowie 55D bis 65D verwendet werden, die im Schaftbereich auch bei dünnwandiger Ausführung, im Verbund mit einem lumenstabilisierenden, wellschlauchartigen Profil, eine ausreichende Stabilität des Lumens sowie eine ausreichend spannungslose Biegbarkeit des Schaftes aufweisen. Beispielsweise kann der ausgeformte Schaft folgende Merkmale aufweisen: Außendurchmesser 3,5 mm, Innendurchmesser 3,2 mm, Polyurethan der Härte Shore 95A, Korrugationsamplitude 0,7 mm, peak-to-peak Abstand der Korrugation 0,5 mm. Der ausgeformte Ballonanteil weist auf: Wandstärke vesikal - 10 µm bei maximal 25 mm Durchmesser, Wandstärke - trans-urethral 20 µm bei maximal 10 mm Durchmesser. Die Befüllung des Ballons erfolgt vorzugsweise durch eine Zuleitung 17, die im Fügebereich des Ballonendes 2b auf dem Schaft, zwischen Schaft und Ballonende eingebracht ist, und hier in den Ballon mündet. Die Zuleitung wird vorzugsweise in eine umhüllende Schlauchfolie 18 gefasst, die am Ballonende 2b anschließt und vorzugsweise über die gesamte Länge des Schaftes reicht.

### Bezugszeichenliste

| | | | |
|---|---|---|---|
| 1 | Vorrichtung | F | Faltung |
| 2 | Schaftkörper | PU | Hüllenende |
| 3 | Ballon | | |
| 4 | vesikales Ballonsegment | | |
| 5 | urethrales Ballonsegment | | |
| 6 | Befüll-Zuleitung | | |
| 7 | Anzeigemechanismus | | |
| 8 | Abschlusselement | | |
| 9 | Öffnung | | |
| 10 | ringartiges Dichtungselement | | |
| 11 | Scheibe | | |
| 12 | Öffnung | | |
| 13 | Hülse | | |
| 14 | Katheterschaft | | |
| 15 | Wellartiges Profil | | |
| 17 | Zuleitung | | |
| 18 | Schlauchfolie | | |
| IV | intra-vesikaler Anteil | | |
| BT | Blasentrigonum | | |
| TU | trans-urethraler Fortsatz | | |
| TM | Trichtermündung | | |
| TMV | Trichter-Versteifungselement | | |
| PH | Prostata-Resektionshöhle | | |
| TP | Ballonhülle | | |
| TPF | Faltenbalgartiges Profil | | |

## Patentansprüche

1. Vorrichtung (1) in Form eines Tubus oder Katheters zum Drainieren oder Verschließen eines natürlichen oder künstlichen Blasenausgangs, umfassend einen von extrakorporal bis in die Blase hinein applizierbaren Schaftkörper (2) sowie einen jenen manschettenförmig umgebenden Ballon (3) mit (i) einem intra-vesikal platzierbaren Ballonsegment (IV) zur vesikalen Verankerung des Schaftkörpers (2) in der Blase, sowie mit (ii) einem urethralen Ballonsegment (TU), das sich zwecks urethraler Dichtung in die Harnröhre hinein oder durch die Harnröhre hindurch erstreckt, **dadurch gekennzeichnet, dass** das urethrale Ballonsegment (TU) mit einem residualen Durchmesser präformiert ist, der den Durchmesser des Harnröhrenlumens um das 0,5- bis 1,5-fache, bevorzugt um das 0,5- bis 1,0-fache, überschreitet.

2. Vorrichtung nach Anspruch 1, **dadurch gekennzeichnet, dass** der Ballon (3)
a) dünnwandig ausgebildet ist mit einer Wandstärke von 5 bis 15 µm; und/oder
b) aus einem dünnwandigen Schlauchmaterial mit einer Wandstärke von 5 bis 15 µm durch Blasformung aus einem vorextrudierten Schlauchmaterial hergestellt ist; und/oder
c) aus Polyurethan besteht; und/oder
d) bereits bei der Herstellung auf sein Arbeitsmaß vorgeformt ist und dem Schaftkörper (2) in situ in nur inkomplett befülltem, schlaffem Zustand aufsitzt; und/oder
e) eine Hülle mit einer geringen Volumendehnbarkeit aufweist.

3. Vorrichtung nach einem der vorhergehenden Ansprüche, **dadurch gekennzeichnet, dass** der Ballon (3) mit einem gasförmigen Medium befüllt oder befüllbar ist, wobei das ankernde intra-vesikale Ballonsegement (IV) und das dichtende urethrale Ballonsegment (TU) des Ballons (3) durch das gasförmige Füllmedium nur unvollständig bzw. partiell befüllt ist, dessen Menge derart bemessen ist, dass der Ballon (3) als Ganzes in einem schlaffen Dehnungszustand verbleibt, die Ballonwandung also keiner permanent wirkenden, dehnenden Kraft ausgesetzt ist.

4. Vorrichtung nach einem der Ansprüche 1 bis 3, **gekennzeichnet durch** eine unmittelbar in das proximale Ende des Ballons (3) mündende Zuleitung für die Einleitung des Füllmediums in den Ballon (3).

5. Vorrichtung nach einem der vorhergehenden Ansprüche, **gekennzeichnet durch** einen Zuleitungs-Spalt zwischen dem proximalen Ende des Ballons (3) und der äußeren Fläche des Schaftkörpers (2) für die Einleitung des Füllmediums in den Ballon (3).

6. Vorrichtung nach einem der vorhergehenden Ansprüche, **gekennzeichnet durch** ein in eine Zuleitung zu dem Ballon (3) eingefügtes, endständiges Einwegeventil, das sich beim Aufsetzen einer Füllspritze öffnet und beim Entfernen des Spritzenkonus selbständig verschließt, und/oder durch eine an eine Zuleitung zu dem Ballon (3) angeschlossene oder anschließbare, manometrisch wirkende Druckanzeige.

7. Vorrichtung nach einem der vorhergehenden Ansprüche, **gekennzeichnet durch** einen Fülldruck innerhalb des Ballons (3) in der Größenordnung von rund 10 mbar bzw. 5 bis 7 mmHg.

8. Vorrichtung nach einem der vorhergehenden Ansprüche, **gekennzeichnet durch** eine antibakterielle Beschichtung auf der Oberfläche des Ballons (3), oder auf der Oberfläche des urethralen Ballonsegmentes (TU).

9. Vorrichtung nach einem der vorhergehenden Ansprüche, **dadurch gekennzeichnet, dass** der Schaftkörper (2) aus Polyurethan (PUR) gefertigt ist, und/oder aus Materialien der Shore-Härten 60A bis 90A, oder aus Materialien des Härtebereiches 70 bis 85A.

10. Vorrichtung nach einem der vorhergehenden Ansprüche, **dadurch gekennzeichnet, dass** der drainierende Schaftkörper (2)
a) einen Außendurchmesser in einem Bereich von 2 mm bis 6 mm aufweist, oder in einem Bereich von 3 mm bis 5 mm; und/oder
b) eine Wandungsstärke in einem Bereich von 0,1 mm bis 0,5 mm, oder in einem Bereich von 0,15 mm bis 0,3 mm.

11. Vorrichtung nach einem der vorhergehenden Ansprüche, **dadurch gekennzeichnet, dass** der Ballon (3) aus einer einzigen, durchgängigen Ballonhülle besteht, wobei ein Ende des Ballons (3) durch das andere gestülpt ist sein kann.

12. Vorrichtung nach einem der Ansprüche 1 bis 10, **dadurch gekennzeichnet, dass** das urethrale Ballonsegment (TU) des Ballons (3) ein oder mehrere Ballonkompartiment(e) aufweist, die sich nach proximal an einen vesikalen Ballon (3) anschließen und aus separat ausgeformten Ballonelementen aufgebaut sind, die sich unmittelbar sequentiell aneinanderreihen.

13. Vorrichtung nach einem der vorhergehenden Ansprüche, **dadurch gekennzeichnet, dass** der im Rahmen der Formung entstehende, ableitende Schaftkörper (2) eine wellige Korrugation aufweist, so dass er eine erhöhte radiale Stabilität und knickfreie Verbiegbarkeit aufweist.

14. Vorrichtung nach einem der vorhergehenden Ansprüche, **dadurch gekennzeichnet, dass** sowohl der Schaftkörper (2) als auch der Ballon (3) aus einem einzigen Materialrohling ausgeformt ist.

15. Vorrichtung nach einem der vorhergehenden Ansprüche, **dadurch gekennzeichnet, dass** der distale Anteil des Ballons (3) derart ausgeformt ist, dass er den Maßen einer Prostata-Resektionshöhle (PH) entspricht oder mit einem diesbezüglich residual dimensionierten Durchmesser versehen ist, während sich proximal ein urethrales Ballonsegment (TU) anschließt, das sich in die Harnröhre hinein erstreckt.

16. Vorrichtung nach einem der vorhergehenden Ansprüche, **dadurch gekennzeichnet, dass** das proximale Ende des urethralen Ballonsegmentes (5) in eine sphärische oder diskoide Erweiterung (PU) des Ballons (3) übergeht, die sich unmittelbar vor der äußeren Harnröhrenöffnung entfaltet.

17. Vorrichtung nach Anspruch 16, **dadurch gekennzeichnet, dass** die sphärische oder diskoide Erweiterung (PU) des Ballons (3) ein auf dem Schaftkörper (2) manschettenförmig aufsitzendes Abschlusselement (8), bspw. in Form einer Scheibe (11), umfasst, welches außen vor der sphärischen oder diskoiden Erweiterung (PU) am proximalen Ende des urethralen Ballonsegmentes (5) sitzt, wobei das Abschlusselement (8) relativ zum Schaftkörper (2) verschiebbar und auf jenem arretierbar ist, so dass eine längenanpassende Stauchung des extrakorporalen Endes zum Orificium hin möglich ist, wobei das verschiebbare Abschlusselement (8) in das prä-urethrale, sphärisch oder diskoid erweiterte Segment (PU) eintaucht und so vor dem direklem Kontakt mit dem empfindlichen Orificium geschützt ist.

## Claims

1. A device (1) in the form of a tube or catheter for draining or sealing a natural or artificial bladder outlet, comprising a shaft body (2) that can be applied extracorporeally into the bladder, as well as a balloon (3) that surrounds it like a cuff with (i) an intra-vesically placeable balloon segment (IV) for the vesical anchoring of the shaft body (2) in the bladder, as well as (ii) a transurethral balloon segment (TU), which extends into the urethra or through the urethra for the purpose of a transurethral sealing, **characterized in that** the transurethral balloon segment (TU) is preformed with a residual diameter, which exceeds the diameter of the urethral lumen by 0.5 to 1.5 times, preferably by 0.5 to 1.0 times.

2. The device according to claim 1, **characterized in that** the balloon (3)
a) is configured with thin walls, having a wall thickness from 5 to 15 µm; and/or
b) is made by blow-molding from a pre-extruded thin-walled tubing, having a wall thickness from 5 to 15 µm, and/or
c) consists of polyurethan; and/or
d) is already pre-shaped to its working dimensions during manufacture and rests upon the shaft body (2) in situ in an only incompletely filled, flaccid state; and/or
e) comprises an envelope having a low volumetric expandability.

3. The device according to one of the preceding claims, **characterized in that** the balloon (3) is or can be filled with a gaseous medium, wherein the anchoring intra-vesical balloon segment (IV) and the sealing urethral balloon segment (TU) of the balloon (3) are only incompletely or, respectively, partially filled with the gaseous filling medium, the amount of which is dimensioned in such a way that the balloon (3) as a whole remains in a flaccid state of expansion, i.e. that the balloon wall is not exposed to a permanent expanding force.

4. The device according to one of the claims from 1 to 3, **characterized by** a feed line opening directly into the proximal end of the balloon (3) for pouring the filling medium into the balloon (3).

5. The device according to one of the preceding claims, **characterized by** a feed line gap between the proximal end of the balloon (3) and the outer surface of the shaft body (2) for conducting the filling medium into the balloon (3).

6. The device according to one of the preceding claims, **characterized by** a terminal one-way valve interlined into a feed line to the balloon (3), which opens when a filling syringe is applied and automatically closes when the syringe cone is removed, and/or by a manometric pressure indicator connected or connectable to the feed line to the balloon (3).

7. The device according to one of the preceding claims, **characterized by** a filling pressure within the balloon (3) on the order of magnitude of approximately 10 mbar or, respectively, 5 to 7 mmHg.

8. The device according to one of the preceding claims, **characterized by** an antibacterial coating on the surface of the balloon (3) or on the surface of the transurethral balloon segment (TU).

9. The device according to one of the preceding claims, **characterized in that** the shaft body (2) is made of polyurethane (PUR) and/or of materials with Shore hardnesses 60A to 90A, or of materials of the hardness range 70 to 85A.

10. The device according to one of the preceding claims, **characterized in that** the draining shaft body (2)
a) has an outer diameter in a range from 2 mm to 6 mm, or in a range from 3 mm to 5 mm; and/or
b) has a wall thickness in a range from 0.1 mm to 0.5 mm, or in a range from 0.15 mm to 0.3 mm.

11. The device according to any preceding claims, **characterized in that** the balloon (3) consists of a single continuous balloon envelope, wherein one end of the balloon (3) can be turned through the other.

12. The device according to one of claims 1 to 10, **characterized in that** the urethral balloon segment (TU) of the balloon (3) comprises one or more balloon compartiment(s) that adjoin a vesical balloon (3) proximally direction and are constructed of separately molded balloon elements, which lign up in a direct succession.

13. The device according to one of the preceding claims, **characterized in that** the discharging shaft body (2) that is produced upon shaping has a wavy corrugation, so that it has radial stability and kink-free flexibility.

14. The device according to one of the preceding claims, **characterized in that** both the shaft body (2) and the balloon (3) are formed from a single material blank.

15. Device according to one of the preceding claims, **characterized in that** the distal portion of the balloon (3) is configured such that it corresponds to the dimensions of a prostate resection cavity (PH) or has a diameter that is residually dimensioned in this regard, while a urethral balloon segment (TU) adjoins proximally, which extends into the urethra.

16. The device according to one of the preceding claims, **characterized in that** the proximal end of the urethral balloon segment (5) transitions into a spherical or discoidal expansion (PU) of the balloon (3), which is deployed directly upstream of the outer urethral opening.

17. The device according to claim 16, **characterized in that** the spherical or discoidal expansion (PU) of the balloon (3) comprises a termination element (8) mounted on the shaft body (2) like a cuff, for example in the form of a disc (11), which is disposed externally at the proximal end of the urethral balloon segment (5), wherein the termination element (8) can be displaced relative to the shaft body (2) and can be arrested on it so that the extracorporeal end can be compressed toward the orifice in a length-adapting way, wherein the displaceable termination element (8) immerges into the pre-urethral spherically or discoidally expanded segment (PU) and is thereby protected from direct contact with the sensitive orifice.

## Revendications

1. Dispositif (1) en forme de tube ou de cathéter de drainage ou d'obturation d'un orifice de sortie naturel ou artificiel de la vessie, comprenant un corps de tige (2) qui peut être appliqué de l'extérieur du corps jusque dans la vessie ainsi qu'un ballonnet (3) entourant en forme de manchette avec (i) un segment de ballonnet qui peut être placé de manière intravésicale (IV) pour l'ancrage vésical du corps de tige (2) dans la vessie, ainsi que (ii) un segment de ballonnet urétral (TU), qui pour une étanchéification urétrale s'étend à l'intérieur de l'urètre ou à travers l'urètre, **caractérisé en ce que** le segment de ballonnet urétral (TU) est préformé d'un diamètre résiduel qui dépasse le diamètre de la lumière urétrale de 0,5 à 1,5 fois, de préférence de 0,5 à 1,0 fois.

2. Dispositif selon la revendication 1, **caractérisé en ce que** le ballonnet (3)
a) est réalisé à paroi mince avec une épaisseur de paroi entre 5 et 15 µm; et/ou
b) est constitué d'un matériau tubulaire à paroi mince d'une épaisseur de paroi de 5 à 15 µm par moulage par soufflage à partir d'un matériau tubulaire pré-extrudé; et/ou
c) est constitué de polyuréthane; et/ou
d) est déjà préformé à sa dimension de travail lors de la fabrication et repose in situ sur le corps de tige (2) à l'état flasque, rempli seulement de manière incomplète; et/ou
e) comporte une enveloppe de faible extensibilité volumique.

3. Dispositif selon l'une des revendications précédentes, **caractérisé en ce que** le ballonnet (3) est rempli ou peut être rempli d'un fluide gazeux, **en ce que** le segment de ballonnet à ancrage intravésical (IV) et le segment de ballonnet à étanchéification urétrale (TU) du ballonnet (3) ne sont qu'incomplètement ou partiellement remplis par le fluide de remplissage gazeux, dont la quantité est calculée de sorte que le ballonnet (3) dans son ensemble reste dans un état de dilatation flasque, la paroi de ballonnet n'étant donc pas soumise à une force de dilatation agissant en permanence.

4. Dispositif selon l'une des revendications 1 à 3, **caractérisé par** une conduite d'alimentation débouchant directement dans l'extrémité proximale du ballonnet (3) pour l'introduction du fluide de remplissage dans le ballonnet (3).

5. Dispositif selon l'une des revendications précédentes, **caractérisé par** une fente d'alimentation entre l'extrémité proximale du ballonnet (3) et la surface extérieure du corps de tige (2) pour l'introduction du fluide de remplissage dans le ballonnet (3).

6. Dispositif selon l'une des revendications précédentes, **caractérisé par** une valve unidirectionnelle terminale, insérée dans une conduite d'alimentation au ballonnet (3), ladite valve unidirectionnelle s'ouvrant à application d'une seringue de remplissage et se fermant automatiquement à retrait du cône de seringue, et/ou par un indicateur de pression à action manométrique raccordé ou pouvant être raccordé à une conduite d'alimentation au ballonnet (3).

7. Dispositif selon l'une des revendications précédentes, **caractérisé par** une pression de remplissage à l'intérieur du ballonnet (3) de l'ordre d'environ 10 mbar ou de 5 à 7 mmHg.

8. Dispositif selon l'une des revendications précédentes, **caractérisé par** un revêtement antibactérien sur la surface du ballonnet (3), ou sur la surface du segment de ballonnet urétral (TU).

9. Dispositif selon l'une des revendications précédentes, **caractérisé en ce que** le corps de tige (2) est fabriqué en polyuréthane (PUR), et/ou dans des matériaux de dureté Shore 60A à 90A, ou dans des matériaux de la plage de dureté de 70 à 85A.

10. Dispositif selon l'une des revendications précédentes, **caractérisé en ce que** le corps de tige (2) drainant
a) présente un diamètre extérieur dans une plage comprise entre 2 mm et 6 mm, ou dans une plage comprise entre 3 mm et 5 mm; et/ou
b) une épaisseur de paroi dans une plage comprise entre 0,1 mm et 0,5 mm, ou dans une plage comprise entre 0,15 mm et 0,3 mm.

11. Dispositif selon l'une des revendications précédentes, **caractérisé en ce que** le ballonnet (3) est constitué d'une enveloppe de ballonnet unique et continue, **en ce qu'**une extrémité du ballonnet (3) peut être glissée dans l'autre.

12. Dispositif selon l'une des revendications 1 à 10, **caractérisé en ce que** le segment de ballonnet urétral (TU) du ballonnet (3) comporte un ou plusieurs compartiments de ballonnet, qui se raccordent de manière proximale à un ballonnet (3) vésical et qui sont constitués d'éléments de ballonnet moulés séparément qui s'alignent directement de manière séquentielle.

13. Dispositif selon l'une des revendications précédentes, **caractérisé en ce que** le corps de tige (2) divergent qui se forme dans le cadre du moulage présente une corrugation ondulée, de sorte qu'il présente une stabilité radiale accrue et une aptitude à la flexion sans pliure.

14. Dispositif selon l'une des revendications précédentes, **caractérisé en ce que** le corps de tige (2) et le ballonnet (3) sont tous deux formés à partir d'une seule ébauche de matériau.

15. Dispositif selon l'une des revendications précédentes, **caractérisé en ce que** la partie distale du ballonnet (3) est formée de telle sorte qu'elle correspond aux dimensions d'une cavité de résection de la prostate (PH) ou qui est dotée d'un diamètre dimensionné de manière résiduelle par rapport à celle-ci, tandis qu'un segment de ballonnet urétral (TU) s'y raccorde de manière proximale et s'étend dans l'urètre.

16. Dispositif selon l'une des revendications précédentes, **caractérisé en ce que** l'extrémité proximale du segment de ballonnet urétral (5) se prolonge par une extension (PU) sphérique ou discoïde du ballonnet (3) qui se déploie immédiatement en amont de l'orifice urétral externe.

17. Dispositif selon la revendication 16, **caractérisé en ce que** l'extension (PU) sphérique ou discoïde du ballonnet (3) comporte un embout (8) en forme de manchette, par exemple en forme de disque (11), reposant sur le corps de tige (2), lequel embout repose à l'extérieur en amont de l'extension (PU) sphérique ou discoïde sur l'extrémité proximale du segment de ballonnet urétral (5), **en ce que** l'embout (8) par rapport au corps de tige (2) peut être déplacé et bloqué sur celui-ci, de sorte qu'une compression adaptable en longueur de l'extrémité extracorporelle à l'orifice est possible, **en ce que** l'embout (8) déplaçable est immergé dans le segment (PU) pré-urétral, étendu de manière sphérique ou discoïde et est ainsi protégé du contact direct avec l'orifice sensible.
